(19) Europäisches Patentamt / European Patent Office / Office européen des brevets

(11) **EP 2 831 556 B1**

(12) **FASCICULE DE BREVET EUROPEEN**

(45) Date de publication et mention
de la délivrance du brevet:
**12.05.2021 Bulletin 2021/19**

(21) Numéro de dépôt: **13719946.9**

(22) Date de dépôt: **29.03.2013**

(51) Int Cl.:
*G01L 25/00* (2006.01)        *G01D 3/02* (2006.01)
*A61B 5/22* (2006.01)

(86) Numéro de dépôt international:
**PCT/FR2013/050694**

(87) Numéro de publication internationale:
**WO 2013/144523 (03.10.2013 Gazette 2013/40)**

(54) **SYSTEME DE MESURE D'UNE FORCE DE PREHENSION PALMAIRE**

SYSTEM ZUR MESSUNG EINER HANDFLÄCHEN-GRIFFKRAFT

SYSTEM FOR MEASURING A PALMAR GRIPPING FORCE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **30.03.2012 FR 1252928**

(43) Date de publication de la demande:
**04.02.2015 Bulletin 2015/06**

(73) Titulaire: **Association Institut de Myologie
75013 Paris (FR)**

(72) Inventeur: **HOGREL, Jean-Yves
F-92120 Montrouge (FR)**

(74) Mandataire: **Gauchet, Fabien Roland et al
Brandon IP
64, rue Tiquetonne
75002 Paris (FR)**

(56) Documents cités:
**US-A- 5 014 229      US-A- 5 398 696
US-A- 5 904 639      US-A1- 2002 132 655**

- **A M Ra ET AL: "Generalised Sensor Linearisation
and Calibration", , 31 October 2004 (2004-10-31),
XP055523908, Retrieved from the Internet:
URL:http://doras.dcu.ie/18153/1/Amra_Pasic .pd
f [retrieved on 2018-11-14]**

## Description

L'invention se rapporte au domaine des procédés et dispositifs de mesure de la force de préhension palmaire pour évaluer l'intégrité de la commande et des effecteurs musculaires, en particulier de l'avant bras et de la main d'un individu.

L'invention fait donc partie du domaine de la métrologie ; elle permet de mesurer des efforts.

Lors de l'évaluation fonctionnelle musculaire d'un individu, la force de préhension palmaire est une des fonctions les plus intéressantes car elle constitue un bon indicateur de la capacité musculaire globale, du statut nutritionnel, de l'autonomie et de façon générale de la santé de l'individu.

On connaît des dispositifs qui permettent de quantifier un état pathologique ou post-traumatique et de valider l'effet d'un traitement sur le système neuromusculaire, en particulier en rééducation.

Dans ce contexte, un besoin s'est développé pour des patients atteints par exemple de pathologies neuromusculaires et dont la force de préhension palmaire est parfois très faible. A ces niveaux de forces, il est important que le dispositif de mesure soit suffisamment sensible et précis pour évaluer de manière fiable la force de préhension palmaire du patient. Il s'agit de mesurer afin de suivre l'évolution des efforts développés par des patients très faibles.

Par ailleurs, il importe que les dispositifs de mesure permettent également de mesurer la force de préhension palmaire de personnes bien portantes, et de sportifs.

## ETAT DE LA TECHNIQUE ANTERIEURE

**En** milieu clinique, l'évaluation de la préhension palmaire est effectuée grâce à des tests manuels par exemple par un kinésithérapeute. Il est connu que ces tests manuels n'apportent pas la précision nécessaire à un suivi rigoureux, notamment dans le cadre d'essais thérapeutiques.

L'art antérieur propose des dispositifs de mesure équipés de capteurs. De façon générale, ces dispositifs ne permettent pas de mesurer avec précision une large gamme de forces et ne sont pas sensibles aux petites forces générées par les patients les plus faibles.

On connaît par exemple un premier groupe de poignées comprenant des ergomètres mécaniques où la réponse des capteurs est déterminée par la déformation d'un ressort ou d'une lame métallique. La lecture des mesures est réalisée le plus souvent à l'aide d'une aiguille sur un cadran ou à l'aide d'un afficheur digital lorsqu'une jauge de contrainte est utilisée pour convertir la déformation de la pièce mécanique en tension électrique. Les inconvénients de ces ergomètres résident dans les défauts de précision, en particulier dans les ergomètres à aiguilles. En outre il est difficile d'enregistrer des efforts à des intensités données car le type d'affichage n'est pas adapté. En effet, la plupart de ces ergomètres ne retient que la valeur maximale de préhension.

On connaît un deuxième groupe de poignées comprenant des ergomètres hydrauliques où la poignée contient un fluide dont la compression entraîne la déformation d'une membrane. L'affichage est assuré par un cadran à aiguilles ou un cadran digital. De même que pour les poignées mécaniques, il est souvent difficile de demander à un sujet de maintenir un niveau d'effort donné, de sorte que les mesures sont difficiles à réaliser.

Un autre groupe de poignées regroupe des systèmes électroniques connectables à un module de traitement de données tel qu'un ordinateur. L'inconvénient principal de ce dernier groupe réside dans des prix élevés notamment en raison de l'utilisation d'une carte d'acquisition et de traitement de données issues du capteur utilisé. En effet, pour obtenir des mesures précises, et fiables, l'acquisition et le traitement de données effectués par ladite carte sont basés sur des processus complexes qui impliquent des temps de calcul importants, une forte consommation d'énergie et des composants multiples et/ou onéreux. En outre, la très grande majorité des systèmes électroniques actuels sont dotés moyens de communication filaire, ce qui présente l'inconvénient d'être peu pratique quand le patient est en fauteuil roulant ou immobilisé sur un lit par exemple.

En outre, la sensibilité et la précision des systèmes existants peuvent se révéler insuffisantes pour les patients les plus faibles.

D'autres dispositifs comprennent des capteurs associés à une méthode de calibration mise en œuvre par des programmes informatiques.

Ainsi, on connaît par le document WO 2011/044520 un système, un procédé et un appareil permettant d'effectuer des exercices isométriques à des fins diagnostiques ou à des fins thérapeutiques. L'appareil divulgué comprend un port de communication externe à un ordinateur permettant notamment de le programmer. L'appareil comprend des capteurs de charge et des moyens d'amplification électroniques associés à un convertisseur analogique/numérique. Ce document divulgue également une calibration de l'appareil par dix neuf combinaisons de six masses standard corrélées à un calcul du type erreur quadratique moyenne.

Ce type de système n'est cependant pas optimal car il comprend une calibration basée sur un calcul complexe ce qui induit des temps de calculs longs et des moyens de calcul onéreux.

Les documents US5904639 et US5398696 décrivent des systèmes de mesure d'une préhension palmaire présentant un arrangement ne permettant pas une évaluation de la préhension palmaire précise.

## EXPOSE DE L'INVENTION

L'invention vise à remédier aux inconvénients de l'état de la technique et notamment à proposer un système de mesure d'une force de préhension palmaire selon la revendication 1.

Ainsi le système de mesure selon l'invention présente une sensibilité très intéressante, jamais atteinte par l'art antérieur. En outre ce système est adaptable sur une large plage de mesures d'où un grand nombre d'applications possibles, depuis les personnes musculo-déficientes jusqu'au sportifs de haut niveau.

De préférence, le calibrage du capteur est réalisé sur au moins trois mesures de calibrage.

Avantageusement, le module électronique comprend en outre des moyens de transmission de ladite valeur de force calibrée vers une unité d'affichage et/ou une unité de stockage et/ou une unité de traitement de données.

De préférence, l'unité d'affichage et/ou l'unité de stockage et/ou l'unité de traitement sont prévues dans le module électronique du dispositif de mesure.

Ainsi, le système selon l'invention permet des mesures améliorées du point de vue de la précision aussi bien pour des patients ayant de très faibles capacités musculaires, que pour des personnes en bonne santé ou des sportifs de haut niveau.

En outre, la résolution est améliorée de manière significative dans un tel système de mesure. En particulier, une résolution de l'ordre de 10g peut être obtenue.

Ainsi il est possible de calibrer le capteur au moyen d'une fonction simplifiée. Une telle simplification de calcul permet d'utiliser des microcontrôleurs moins complexes que ceux de l'art antérieur. Il en résulte une baisse significative des coûts et des dépenses d'énergie. En outre des microcontrôleurs de petite taille peuvent être utilisés.

Selon une variante préférée, l'unité d'affichage et/ou l'unité de stockage et/ou l'unité de traitement et/ou l'unité de transmission du signal sont prévues dans un module spécifique connecté audit module électronique du dispositif de mesure, ladite transmission étant filaire et/ou sans fil.

Par exemple l'affichage et/ou le stockage des données sont réalisés sur un ordinateur, ce qui permet de prévoir des logiciels scientifiques et/ou ludiques pour l'acquisition et la visualisation des signaux mesurés. Cette caractéristique permet d'imaginer un grand nombre de protocoles différents d'évaluation, de rééducation ou d'entraînement.

Préférentiellement le calibrage du capteur est réalisé sur au moins trois mesures de calibrage.

Avantageusement, la poignée du dispositif de mesure comprend au moins une barre interne parallèle à un premier élément du cadre externe et mobile en translation vis-à-vis dudit premier élément du cadre externe ; le capteur de force est configuré pour mesurer une force associée à ladite translation.

Selon une variante, la poignée comprend un moyen de réglage d'un écart initial entre la barre interne mobile et le premier élément du cadre externe.

De préférence, le système de mesure comprend des moyens de sélection d'un mode de fonctionnement choisi parmi :

- un mode dit de mesure pour ladite mesure d'une force de préhension palmaire,
- un mode dit de calibration pour la mise en œuvre dudit calibrage.

Selon un aspect intéressant, le système de mesure comprend des moyens de connexion filaire et/ou sans fil à une unité de commande.

Un autre objet de l'invention consiste en un produit-programme informatique chargeable dans une mémoire d'un ordinateur et/ou du microcontrôleur du module électronique, comprenant des parties de code logiciel pour réaliser des étapes de calibrage et de mesure d'un système de mesure de force de préhension palmaire tel que décrit précédemment.

## BREVE DESCRIPTION DES FIGURES

D'autres caractéristiques, détails et avantages de l'invention ressortiront à la lecture de la description qui suit, en référence aux figures annexées, qui illustrent :

- les figures 1A et 1B, un dispositif selon une première variante de l'invention configuré pour un système selon l'invention ;
- la figure 2, un schéma du module électronique pour un dispositif selon l'invention ;
- la figure 3, une représentation graphique de la calibration du capteur.

Pour plus de clarté, les éléments identiques ou similaires sont repérés par des signes de référence identiques sur l'ensemble des figures.

## DESCRIPTION DETAILLEE DES MODES DE REALISATION

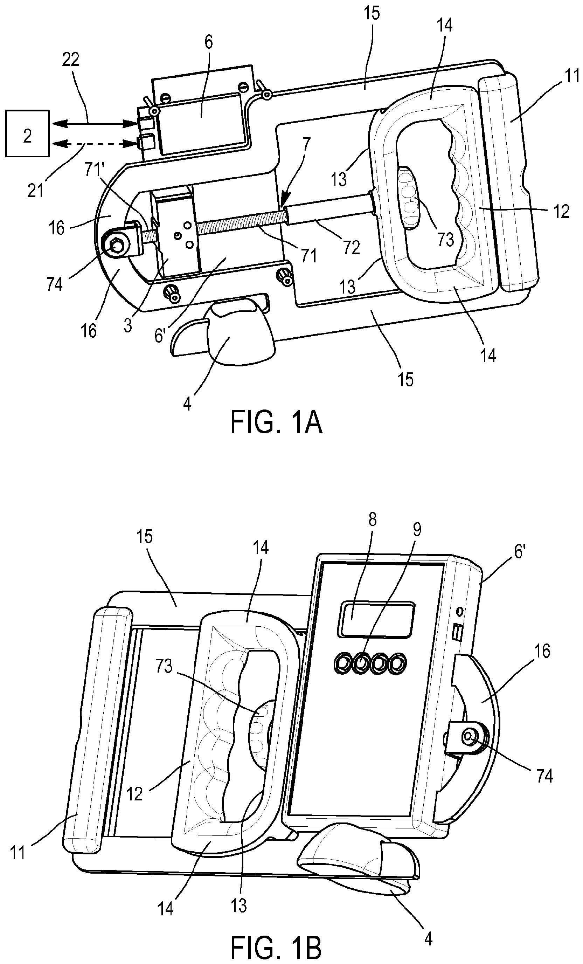

Selon les représentations des figures 1A et 1B, le dispositif 1 selon une première variante de l'invention comprend un cadre externe formé par deux barres latérales 15 et une barre supérieure 11. Le dispositif comprend en outre un cadre interne formé par deux barres latérales 14 juxtaposées guidées et parallèles aux barres 15 ; une barre supérieure 12 est disposée parallèlement à la barre 11. Le cadre interne comprend également une barre inférieure 13. Le cadre interne 12,13,14 présente globalement une forme rectangulaire; la barre supérieure 12 sera en prise avec la main de l'utilisateur qui maintiendra simultanément la barre supérieure 11. Le cadre interne peut être monobloc, obtenu par exemple par injection plastique moulée.

En outre, de façon optionnelle, une barre latérale 15 du cadre externe peut comprendre un pied 4 permettant de poser la poignée sur une surface plane.

Un circuit électronique 6' comprenant un module électronique est fixé sur le cadre externe. Un capteur 3 constitué d'une jauge de contrainte à haute précision, est disposé contre le circuit électronique 6', et est attaché par un axe 7 parallèle aux barres 15 et lui-même fixé à la barre 13 du cadre interne. Ainsi, ce capteur 3 est solidaire du cadre interne, et est connecté à un module électronique 6. Comme il sera expliqué ci-après, cet arrangement permet de mesurer les efforts liés aux déplacements de la barre supérieure 12 du cadre interne vers la barre supérieure 11 du cadre externe.

La poignée comprend en outre un moyen de réglage de l'ouverture de la poignée, en particulier de l'écart initial entre les barres 11 et 12 maintenues et actionnées par l'utilisateur. Ce moyen de réglage est ici constitué d'un axe fileté 71 coopérant avec une partie taraudée 72. L'axe fileté 71 est vissé sur le capteur 3 sur une première face, tandis que la partie taraudée 72 est fixée au cadre interne 12,13,14. Une molette de réglage 73 est prévue ici au niveau de la barre 13 afin de régler l'écart initial entre le cadre interne et le cadre externe. La figure 1A représente le cadre interne en contact avec le cadre externe tandis que la figure 1B représente le cadre interne en position éloignée du cadre externe. Un deuxième axe 71' aligné avec l'axe 71 vient s'attacher de l'autre côté du capteur.

Pour plus de stabilité et un meilleur équilibrage des forces, l'axe 7 de la poignée en particulier l'axe interne fileté 71 est fixé au cadre externe au milieu d'une barre 16 opposée à la barre supérieure 11. Un moyen de fixation 74 ici constitué d'un système de vis à pivot permet un libre mouvement de l'axe 71' du capteur 3.

Le capteur utilisé est un capteur SML Low Height Load Cell commercialisé par la société Interface Inc. Le capteur préféré présente une capacité nominale d'environ 89kgf (200 lbf).

En se référant à la figure 1B, le circuit électronique 6', le capteur 3 et une partie de l'axe interne fileté 71 sont logés dans un boîtier qui présente des touches 9 et un écran par exemple de type LCD 8. Les touches 9 servent par exemple à mettre le dispositif en mode calibrage ou en mode mesure et l'affichage 8 permet de visualiser directement les forces mesurées. Ainsi cette variante peut être utilisée de manière autonome, c'est-à-dire sans connexion à un ordinateur ou tout autre dispositif de stockage et de traitement de données.

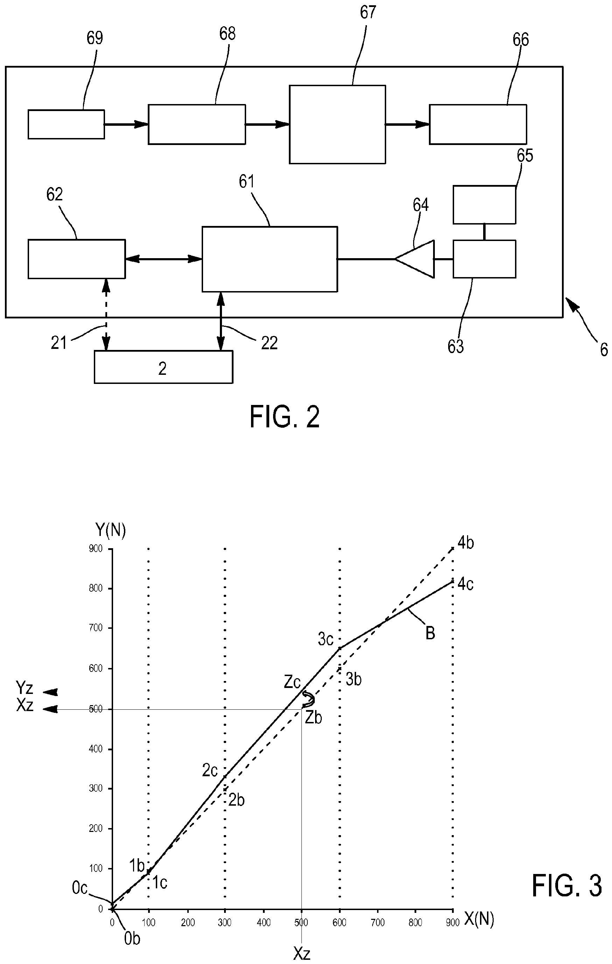

Comme illustré sur la figure 2, le module électronique 6 est connectable à un ordinateur 2. Des moyens de connexions filaires 22 tels qu'un câble RS 232 peuvent être utilisés à cette fin. Des moyens de connexion sans-fil 21 tels qu'un émetteur radiofréquence 62 sont de préférence utilisés. Ainsi le dispositif selon l'invention peut coopérer avec un programme informatique mettant en œuvre l'affichage et le traitement des informations reçues dudit capteur de force 3. Une interface ludique peut être implémentée sans sortir du cadre de l'invention.

En se référant à la figure 2, le module électronique 6 comprend notamment des sous modules 66,67 destinés à l'alimentation et à la recharge électrique du dispositif. A titre illustratif on peut voir sur la figure 2, un sous-module dit « power jack » 69 pour la connexion à une source d'alimentation en courant externe au dispositif 1, un sous-module circuit de charge 68, un sous-module batteries 67 et un sous-module régulation et alimentation 66.

Le module électronique 6 comprend également des sous modules tels que : un sous-module dit « Ref. tension » 65 destiné à fixer une tension de référence des mesures ; un sous-module jauge de contrainte 63 ; un sous module d'amplification de tension 64 ; un sous module microcontrôleur 61.

Le microcontrôleur 61 comprend avantageusement un EPROM.

Le microcontrôleur 61 est connectable à l'ordinateur 2 au moyen d'une connexion filaire 22 par l'intermédiaire d'un port série. L'émetteur 62 permet aussi d'envoyer des informations par exemple à l'ordinateur 2, tel que symbolisé par la référence 21 (liaison sans fil).

Le module microcontrôleur 61 intègre plusieurs composants et notamment une RAM, une mémoire FLASH et un convertisseur analogique numérique. Ces composants sont configurés de sorte à permettre une calibration des mesures des forces appliquées à la jauge de contrainte 63 par l'intermédiaire du capteur 3 du dispositif selon l'invention. A cet effet, un programme informatique est chargé dans la mémoire du microcontrôleur 61 de sorte à réaliser ladite calibration et lesdites mesures selon un procédé donné.

La connexion de préférence sans fil à un ordinateur présente un intérêt particulier dans l'utilisation de la poignée.

Par exemple, un logiciel peut être installé sur un ordinateur (en combinaison avec un récepteur sans fil adapté), ce qui permet de visualiser en temps réel l'acquisition des forces mesurées, la comparaison avec des mesures déjà acquises etc. Le patient ou le sportif testé peut ainsi suivre en temps réel sa progression. Ainsi lors de tests, l'attention du patient est focalisée sur ces illustrations ce qui est avantageux lors de tests d'évaluation, notamment avec des enfants.

En outre l'utilisation de tels logiciels permet d'assurer un meilleur suivi des mesures, d'effectuer des statistiques de manière plus aisée, et ainsi d'aller plus loin dans l'analyse des données.

La figure 3 illustre la calibration du capteur 3 dans le système selon l'invention. Bien entendu, l'illustration graphique a pour but d'expliquer les calculs faits par le système de mesure. Comme on peut le voir sur cette figure, cinq mesures brutes ont été effectuées à 0, 100, 300, 600 et 900 N. La répartition des mesures brutes sur le repère de la figure 3 est représentée par la courbe d'étalonnage B en trait plein. Chaque point d'étalonnage 0c à 4c est réalisé en associant en abscisse la mesure brute X0 à X4 du capteur et en ordonnée la valeur réelle Y0 à Y4 de la force de calibration correspondante. Les points de mesures brutes 0b à 4b ont la même abscisse et la même ordonnée, correspondant à la mesure brute (sans calibration).

Ainsi, afin de compenser les défauts de linéarité du capteur, il est possible de définir une courbe de calibration qui permet d'afficher sur la poignée une mesure corrigée Yz de la mesure brute Xz enregistrée par le capteur.

Il est intéressant de souligner que la calibration est réalisée à l'aide de masses certifiées à 0,001g près. Ceci induit une justesse extrême des points de calibration.

Le principe consiste à relever les valeurs brutes enregistrées par le capteur lorsque l'on applique des masses dont la valeur est connue. Afin d'établir cette courbe, il faut avantageusement effacer toute calibration précédente afin que la poignée affiche les valeurs brutes enregistrées par le capteur.

Cette courbe de calibration est définie en mesurant cinq points d'étalonnage : X0, Y0 ; X1, Y1 ; X2, Y2 ; X3, Y3 ; X4, Y4.

Pour chaque masse connue appliquée sur la poignée, la mesure brute du capteur non corrigée 0b à 4b et affichée à l'écran est enregistrée et correspond à la coordonnée X (X0 à X4). La mesure vraie connue de la masse appliquée correspond à la coordonnée Y (Y0 à Y4). Sur la figure 3, la valeur X correspond à une valeur enregistrée par le capteur à une masse donnée. La projection sur la courbe de calibration en trait plein permet d'obtenir la valeur Y qui est la valeur affichée par le capteur après calibration.

Les cinq points de calibration mesurés sont alors utilisés pour établir une courbe par segment. Sur chaque segment, pour une valeur de X enregistrée par le capteur, correspond une valeur corrigée proche des valeurs de références.

Une fois cette courbe de calibration établie et enregistrée dans le système de traitement de données, lorsqu'une masse inconnue Z est appliquée sur la poignée, le capteur enregistre une mesure brute Xz qui est corrigée en fonction du segment sur lequel elle se trouve selon la formule Y'=a X' +b. La mesure affichée par le moyen d'affichage 8 est alors la coordonnée Yz qui correspond à la mesure corrigée de la masse Z. Le point Zc est le point de mesure après calibration, alors que le point Zb correspond à une mesure brute. L'ordonnée Yz du point Zc correspond donc à une mesure corrigée, c'est-à-dire une mesure calibrée.

En variante, les points d'étalonnage 0 et 4 sont imposés à 0 N et 890 N. Le choix des points d'étalonnage 1, 2 et 3 doit être judicieusement effectué en fonction de la gamme d'utilisation de la poignée.

Il est recommandé de choisir les points 100, 300 et 500N pour une poignée adulte et 100, 200 et 300 N pour une poignée enfant.

En pratique la procédure d'étalonnage est la suivante :

Après le lancement du logiciel d'acquisition, et l'effacement d'un éventuel étalonnage précédent, on règle la largeur de la poignée, de préférence sur un cran intermédiaire. On met ensuite la poignée en mode étalonnage.

Un point d'étalonnage « 0 » est de préférence la mesure du capteur lorsque la poignée est posée à plat sans charge. Pour les autres points la poignée est suspendue à une barre fixe horizontale par l'intermédiaire de deux sangles. En variante, on peut prévoir un anneau dans la structure de la poignée dans lequel on insère un crochet pour la suspension de la poignée.

De préférence, on vérifie que l'axe longitudinal de la poignée est vertical, à l'aide d'un niveau à bulle.

Un deuxième point d'étalonnage « 0 » est enregistré lorsque la poignée est suspendue. Trois autres points d'étalonnage sont choisis avec des masses correspondantes.

Toutes les mesures obtenues sont associées à une abscisse X et les valeurs réelles sont associées à une ordonnée Y. On obtient ainsi une courbe de calibration qui est linéaire par segments.

Il est également possible de vérifier que les valeurs fournies par la poignée sont justes. Pour cela on applique sur la poignée des poids étalons de masse connue et on compare les valeurs de référence avec les valeurs affichées par la poignée.

Pour ce faire on refait les étapes exposées précédemment sur un capteur calibré avec des masses étalons de 500g à 60Kg. Une fois les mesures relevées, les valeurs de force « poignée à plat » sont calculées selon la formule suivante :

$$\text{Mesure poignée à plat x Kg} = \text{Mesure poignée suspendue x Kg} - \text{Mesure poignée suspendue 0Kg} + \text{Mesure poignée à plat 0Kg}$$

Les valeurs obtenues sont ensuite comparées aux valeurs de référence.

Si la mesure délivrée par le capteur n'est pas sensiblement linéaire ou si elle présente un léger décalage par rapport aux valeurs de références, il faut alors effectuer une nouvelle calibration de la poignée.

L'utilisation du système selon l'invention a permis de déterminer de manière sensiblement exacte l'évolution des conditions musculaires de certains patients. A titre illustratif, des résultats prouvant les performances du dispositif ont été obtenus chez des patients atteints de dystrophie musculaire de Duchenne ou d'amyotrophie spinale pour lesquels des forces maximales de préhension inférieures à quelques centaines de grammes ont pu être enregistrées. En outre, la répétabilité des mesures a été prouvée dans plusieurs populations d'adultes et d'enfants, sains ou malades.

De nombreuses combinaisons peuvent être envisagées sans sortir du cadre de l'invention telle que revendiquée.

Les valeurs obtenues sont ensuite comparées aux valeurs de référence.

Si la mesure délivrée par le capteur n'est pas sensiblement linéaire ou si elle présente un léger décalage par rapport aux valeurs de références, il faut alors effectuer une nouvelle calibration de la poignée.

L'utilisation du système selon l'invention a permis de déterminer de manière sensiblement exacte l'évolution des conditions musculaires de certains patients. A titre illustratif, des résultats prouvant les performances du dispositif ont été obtenus chez des patients atteints de dystrophie musculaire de Duchenne ou d'amyotrophie spinale pour lesquels des forces maximales de préhension inférieures à quelques centaines de grammes ont pu être enregistrées. En outre, la répétabilité des mesures a été prouvée dans plusieurs populations d'adultes et d'enfants, sains ou malades.

De nombreuses combinaisons peuvent être envisagées sans sortir du cadre de l'invention ; l'homme de métier choisira l'une ou l'autre en fonction des contraintes économiques, ergonomiques, dimensionnelles ou autres qu'il devra respecter.

**Revendications**

1. Système de mesure d'une force de préhension palmaire comprenant un dispositif (1) de mesure de ladite force de préhension palmaire qui comprend

   - une poignée configurée pour recevoir une force de préhension palmaire, la poignée comprenant un cadre externe formé de plusieurs éléments (11,15,16) dont deux barres latérales 15,
   - un capteur de force (3) configuré pour mesurer ladite force de préhension palmaire,
   - un module électronique (6) intégré dans le dispositif (1), fixé sur le cadre externe et comprenant au moins un microcontrôleur (61) connecté au capteur (3) et apte à traiter des données issues dudit capteur (3) disposé contre ledit module électronique,

   **caractérisé en ce que** la poignée comporte un cadre interne (12,13,14) comportant deux barres latérales (14) juxtaposées guidées et parallèles aux barres latérales (15) du cadre externe, et le système comprend un axe (7,71,72) reliant le capteur de force (3) au cadre interne et une jauge de contrainte (63) apte à détecter des efforts depuis une valeur nulle ou quasi nulle, et qui présente une précision d'au minimum d'environ 50g pour une plage de mesures allant de 0 à 90kg, et/ou une sensibilité inférieure à environ 10 g,

   et **en ce que** le module électronique (6) comprend des moyens aptes à réaliser les actions suivantes :

   (a)- calibrer le capteur (3), dans laquelle :

   (a1)- le capteur (3) reçoit une pluralité de forces de calibrage (F0,F1,F2,F3,F4),
   (a2)- établir une fonction de calibration linéaire (D) à partir de points de calibration (0c,1c,2c,3c,4c) dont une première coordonnée est une mesure de capteur (X0-X4) et une deuxième coordonnée (Y0-Y4) est la valeur de ladite force de calibrage, ladite fonction de calibration étant linéaire par segments,

   (b)- enregistrer une valeur de force de préhension palmaire selon une première coordonnée (Xz) d'un point de la fonction de calibration linéaire (D), et déterminer une valeur de force calibrée correspondant à la deuxième coordonné (Yz) dudit point de la fonction de calibration linéaire (D).

2. Système de mesure selon la revendication 1, **caractérisé en ce que** le module électronique (6) comprend des moyens de transmission de ladite valeur de force calibrée vers une unité d'affichage (8) et/ou une unité de stockage et/ou une unité de traitement de données.

3. Système de mesure selon la revendication 2, **caractérisé en ce que** l'unité d'affichage et/ou l'unité de stockage et/ou l'unité de traitement sont prévues dans le module électronique (6) du dispositif de mesure.

4. Système de mesure selon la revendication 2 ou 3, **caractérisé en ce que** l'unité d'affichage et/ou l'unité de stockage et/ou l'unité de traitement sont prévues dans un module spécifique connecté audit module électronique du dispositif de mesure, ladite transmission étant filaire et/ou sans fil.

5. Système de mesure selon l'une des revendications 1 à 4, **caractérisé en ce que** le calibrage du capteur (3) est réalisé sur au moins trois mesures de calibrage.

6. Système de mesure selon l'une des revendications 1 à 5, **caractérisé en ce que** la poignée du dispositif de mesure comprend au moins une barre interne (12) parallèle à un premier élément (11) du cadre externe et mobile en translation vers ledit premier élément du cadre externe, et **en ce que** le capteur de force (3) est configuré pour mesurer une force associée à ladite translation.

7. Système de mesure selon l'une des revendications 1 à 6, **caractérisé en ce que** la poignée comprend un moyen de réglage (7) d'un écart initial entre la barre interne mobile (12) et le premier élément (11) du cadre externe.

8. Système de mesure selon l'une des revendications 1 à 7, **caractérisé en ce qu'**il comprend des moyens de sélection (9) d'un mode de fonctionnement choisi parmi :

   - un mode dit de mesure pour ladite mesure d'une force de préhension palmaire,
   - un mode dit de calibration pour la mise en œuvre de ladite calibration.

9. Système de mesure selon l'une des revendications 1 à 8, **caractérisé en ce qu'**il comprend des moyens de connexion filaire et/ou sans fil à une unité de commande

10. Produit-programme informatique chargeable dans une mémoire d'un ordinateur et/ou du microcontrôleur du module électronique du système de mesure selon l'une quelconque des revendications précédentes, comprenant des parties de code logiciel pour réaliser des étapes de calibrage et de mesure dudit système de mesure.

**Patentansprüche**

1. System zum Messen einer Handflächen-Griffkraft, umfassend eine Vorrichtung (1) zum Messen der Handflächen-Griffkraft, die umfasst

   - einen Griff, der konfiguriert ist, um eine Handflächen-Griffkraft zu empfangen, wobei der Griff einen äußeren Rahmen umfasst, der aus mehreren Elementen (11, 15, 16), darunter zwei Seitenleisten 15 gebildet ist,
   - einen Kraftsensor (3), der konfiguriert ist, um die Handflächen-Griffkraft zu messen,
   - ein in der Vorrichtung (1) integriertes Elektronikmodul (6), das an dem äußeren Rahmen befestigt ist und mindestens einen Mikrocontroller (61) umfasst, der mit dem Sensor (3) verbunden ist, und imstande ist, aus dem Sensor (3), der an dem Elektronikmodul angeordnet ist, stammende Daten zu verarbeiten,

   **dadurch gekennzeichnet, dass** der Griff einen inneren Rahmen (12, 13, 14) beinhaltet, der zwei geführte nebeneinandergestellte, und zu den Seitenleisten (15) des äußeren Rahmens parallele Seitenleisten (14) beinhaltet, und das System eine Achse (7, 71, 72) umfasst, die den Kraftsensor (3) an den inneren Rahmen anschließt, und einen Dehnmessstreifen (63), der imstande ist, Kräfte ab einem Wert Null und quasi Null zu erkennen, und der eine Präzision von mindestens etwa 50 g bei einem Messbereich aufweist, der von 0 bis 90 kg reicht, und/oder eine Empfindlichkeit kleiner als etwa 10 g, und dadurch, dass das Elektronikmodul (6) Mittel umfasst, die imstande sind, die folgenden Aktionen durchzuführen:

   (a)- Kalibrieren des Sensors (3), bei dem:

      (a1) - der Sensor (3) eine Vielzahl von Kalibrierkräften (F0, F1, F2, F3, F4) empfängt,

(a2)- Aufbauen einer linearen Kalibrierfunktion (D) ausgehend von Kalibrierpunkten (0c, 1c, 2c, 3c, 4c), von denen eine erste Koordinate eine Sensormessung (X0-X4) ist und eine zweite Koordinate (Y0-Y4) der Wert der Kalibrierkraft ist, wobei die Kalibrierfunktion segmentiert linear ist,

(b)- Speichern eines Handflächen-Griffkraft entlang einer ersten Koordinate ($X_z$) eines Punkts der linearen Kalibrierfunktion (D),

und Bestimmen eines kalibrierten Kraftwerts entsprechend der zweiten Koordinate ($Y_z$) des Punkts der linearen Kalibrierfunktion (D).

2. System zum Messen nach Anspruch 1, **dadurch gekennzeichnet, dass** das Elektronikmodul (6) Mittel zum Übertragen des kalibrierten Kraftwerts zu einer Anzeigeeinheit (8) und/oder einer Speichereinheit und/oder einer Datenverarbeitungseinheit umfasst.

3. System zum Messen nach Anspruch 2, **dadurch gekennzeichnet, dass** die Anzeigeeinheit und/oder Speichereinheit und/oder Datenverarbeitungseinheit in dem Elektronikmodul (6) der Vorrichtung zum Messen vorgesehen sind.

4. System zum Messen nach Anspruch 2 oder 3, **dadurch gekennzeichnet, dass** die Anzeigeeinheit und/oder Speichereinheit und/oder Datenverarbeitungseinheit in einem speziellen Modul vorgesehen sind, das mit dem Elektronikmodul der Vorrichtung zum Messen verbunden ist, wobei die Übertragung per Draht und/oder drahtlos erfolgt.

5. System zum Messen nach einem der Ansprüche 1 bis 4, **dadurch gekennzeichnet, dass** das Kalibrieren des Sensors (3) anhand mindestens drei Kalibriermessungen durchgeführt wird.

6. System zum Messen nach einem der Ansprüche 1 bis 5, **dadurch gekennzeichnet, dass** der Griff der Vorrichtung zum Messen mindestens eine innere Leiste (12) parallel zu einem ersten Element (11) des äußeren Rahmens und translationsbeweglich zum ersten Element des äußeren Rahmens ist, und dadurch, dass der Kraftsensor (3) konfiguriert ist, um eine Kraft zu messen, die der Translation zugeordnet ist.

7. System zum Messen nach einem der Ansprüche 1 bis 6, **dadurch gekennzeichnet, dass** der Griff ein Einstellmittel (7) einer ursprünglichen Abweichung zwischen der beweglichen inneren Leiste (12) und dem ersten Element (11) des äußeren Rahmens umfasst.

8. System zum Messen nach einem der Ansprüche 1 bis 7, **dadurch gekennzeichnet, dass** es Auswahlmittel (9) eines Betriebsmodus umfasst, ausgewählt aus:

   - einen sogenannten Messmodus für die Messung einer Handflächen-Griffkraft,
   - einen sogenannten Kalibriermodus zum Realisieren der Kalibration.

9. System zum Messen nach einem der Ansprüche 1 bis 8, **dadurch gekennzeichnet, dass** es Mittel zum verdrahteten und/oder drahtlosen Verbinden mit einer Steuereinheit umfasst.

10. Computerprogrammprodukt, das in einen Speicher eines Computers und/oder des Mikrocontrollers des Elektronikmoduls des Systems zum Messen nach einem der vorstehenden Ansprüche ladbar ist, das Teile eines Softwarecodes zum Durchführen der Kalibrier- und Messschritte des Systems zum Messen umfasst.

**Claims**

1. A system for measuring a palmar gripping force comprising a device (1) for measuring said palmar gripping force which comprises

   - a handle configured to receive a palmar gripping force, the handle comprising an outer frame formed by several elements (11, 15, 16) including two lateral bars 15,
   - a force sensor (3) configured to measure said palmar gripping force,
   - an electronic module (6) integrated in the device (1), fastened on the outer frame and comprising at least one microcontroller (61) connected to the sensor (3) and adapted to process data originating from said sensor (3) disposed against said electronic module,

**characterized in that** the handle includes an inner frame (12, 13, 14) including two juxtaposed lateral bars (14) guided and parallel to the lateral bars (15) of the outer frame, and the system comprises an axis (7, 71, 72) linking the force sensor (3) to the inner frame and a strain gauge (63) adapted to detect efforts from a zero or almost zero value, and which has a minimum accuracy of about 50g for a measurement range from 0 to 90kg, and/or a sensitivity lower than about 10 g,
and **in that** the electronic module (6) comprises means adapted to carry out the following actions:

(a)- calibrating the sensor (3), wherein:

(a1)- the sensor (3) receives a plurality of calibration forces (F0, F1, F2, F3, F4),
(a2)- establishing a linear calibration function (D) from calibration points (0c, 1c, 2c, 3c, 4c) where a first coordinate is a sensor measurement (X0-X4) and a second coordinate (Y0-Y4) is the value of said calibration force, said calibration function being piecewise linear,

(b)- recording a palmar gripping force value according to a first coordinate ($X_z$) of a point of the linear calibration function (D), and determine a calibrated force value corresponding to the second coordinate ($Y_z$) of said point of the linear calibration function (D).

2. The measuring system according to claim 1, **characterized in that** the electronic module (6) comprises means for transmitting said calibrated force value towards a display unit (8) and/or a storage unit and/or a data processing unit.

3. The measuring system according to claim 2, **characterized in that** the display unit and/or the storage unit and/or the processing unit are provided in the electronic module (6) of the measuring device.

4. The measuring system according to claim 2 or 3, **characterized in that** the display unit and/or the storage unit and/or the processing unit are provided in a specific module connected to said electronic module of the measuring device, said transmission being wired or wireless.

5. The measuring system according to any of claims 1 to 4, **characterized in that** the calibration of the sensor (3) is carried out over at least three calibration measurements.

6. The measuring system according to any of claims 1 to 5, **characterized in that** the handle of the measuring device comprises at least one inner bar (12) parallel to a first element (11) of the outer frame and movable in translation towards said first element of the outer frame and **in that** the force sensor (3) is configured to measure a force associated to said translation.

7. The measuring system according to any of claims 1 to 6, **characterized in that** the handle comprises a means (7) for setting an initial spacing between the movable inner bar (12) and the first element (11) of the outer frame.

8. The measuring system according to any of claims 1 to 7, **characterized in that** it comprises means (9) for selecting an operating mode chosen amongst:

- a so-called measurement mode for said measurement of a palmar gripping force,
- a so-called calibration mode for the implementation of said calibration.

9. The measuring system according to any of claims 1 to 8, **characterized in that** it comprises means for wired or wireless connection to a control unit.

10. A computer program product loadable in a memory of a computer and/or of the microcontroller of the electronic module of the measuring system according to any one of the preceding claims, comprising software code portions to carry out calibration and measurement steps of said measuring system.

FIG. 1A

FIG. 1B

FIG. 2

FIG. 3

**EP 2 831 556 B1**

## RÉFÉRENCES CITÉES DANS LA DESCRIPTION

### Documents brevets cités dans la description

- WO 2011044520 A
- US 5904639 A
- US 5398696 A